Europäisches Patentamt

European Patent Office

Office européen des brevets

(19)

(11) Publication number: **0 521 897 B1**

# EUROPEAN PATENT SPECIFICATION

(12)

(45) Date of publication of patent specification: **28.06.95**   (51) Int. Cl.⁶: **A61K 39/00**, C12N 5/08

(21) Application number: **91905685.3**

(22) Date of filing: **13.03.91**

(86) International application number:
**PCT/US91/01683**

(87) International publication number:
**WO 91/13632 (19.09.91 91/22)**

(54) **IDIOTYPIC VACCINATION AGAINST B CELL LYMPHOMA.**

(30) Priority: **14.03.90 US 493511**

(43) Date of publication of application:
**13.01.93 Bulletin 93/02**

(45) Publication of the grant of the patent:
**28.06.95 Bulletin 95/26**

(84) Designated Contracting States:
**AT BE CH DE DK ES FR GB GR IT LI LU NL SE**

(56) References cited:

**PROCEEDINGS OF THE NATL. ACADEMY OF SCIENCES USA, vol. 82, December 1985, Washington, DC (US); M. FRANCOTTE et al., pp. 8149-8152/**

**JOURNAL OF IMMUNOLOGY, vol. 133, no. 1, July 1984, Baltimore, MD (US); K. THIELE-MANS et al., pp. 495-501/**

(73) Proprietor: **THE IMMUNE RESPONSE CORPO-RATION**
**5935 Darwin Court**
**Carlsbad, CA 92008 (US)**

(72) Inventor: **BOHLEN, Heribert**
**Laarbeeklaan 103**
**B-1090 Brussels (BE)**
Inventor: **URBAIN, Jacques**
**Tienne du Peuthy 1**
**B-1338 Lasne (BE)**
Inventor: **VAN CAMP, Benjamin**
**Laarbeeklaan 103**
**B-1090 Brussels (BE)**
Inventor: **THIELEMANS, Kristiaan**
**Laarbeeklaan 103**
**B-1090 Brussels (BE)**

(74) Representative: **UEXKÜLL & STOLBERG Paten-tanwälte**
**Beselerstrasse 4**
**D-22607 Hamburg (DE)**

## Description

BACKGROUND OF THE INVENTION

The vertebrate immune system functions to recognize and eliminate materials, such as pathogens, bacteria and viruses, which are recognized as foreign to the host. In addition, the immune system also serves as a surveillance system to eliminate malignant cells, which, because they express altered proteins on the cell surface, are regarded as foreign. Immunological responses to foreign substances, termed antigens, comprise a humoral response and a cellular response. The humoral response involves the production of specific antibodies, or immunoglobulins, which recognize and bind to the antigen. The cellular response involves the proliferation of cells which aid in elimination of the antigens.

An immune response can often be induced or heightened by active immunization with a vaccine (or immunogen) comprising an antigen, or a molecule resembling an antigen. Often it is necessary to provide immune enhancers, termed adjuvants, in addition to the immunogen.

This invention relates generally to the area of active immunization and more specifically to vaccines useful for immunization against lymphomas and to adjuvants useful for changing the magnitude and character of the immune response.

The majority of B lymphoid tumors are characterized by the expression of immunoglobulin (Ig) on the cell membrane. The idiotype (id) of the surface Ig can be regarded as a tumor specific antigen or marker, and has been used as a target for immunotherapy. Monoclonal anti-id antibodies have been used to study the immunobiology of these tumors and have been used in therapeutic trials as well. The passive administration of anti-id monoclonal antibodies (Mabs) especially of mouse origin has been hampered by a number of problems, however, which have reduced their applicability in clinical usage. Some of these problems are (i) the free antigen in circulation (ii), the emerging immune response against mouse Ig and (iii) the heterogeneity of the tumor cells. Several studies have reported that immunization with idiotype protein or subfragments can protect the animal against the outgrowth of a plasmacytoma or surface Ig bearing lymphoma. These studies are reported in, for example, Thielemans et al., J. Exp. Med. 162:19-34 (1985), Lynch et al., Immunol. Rev. 48:45-80 (1980), Hannestad et al., Proc. Natl. Acad. Sci. (USA) 69:2295-2299 (1972), Jorgensen et al., Scand. J. Immunol. 11:29-35 (1980), George et al., J. Immunol. 138:628-634 (1987), Campbell et al., J. Immunol. 139:2825-2833 (1987). The induced immune response includes anti-idiotypic antibodies as well as T cell dependent immunity. In order to evoke such antibodies and immunity against a challenge with tumor cells, it has been shown repeatedly that it is necessary to couple the idiotype protein to a strong immunogenic carrier and to present this conjugate in the presence of a strong adjuvant.

Idiotype heterogeneity has been disclosed during a clinical trial with anti-idiotype antibodies. After an initial partial response induced by she monoclonal antibody, idiotype variant tumor cells emerged at the original tumor-site. It is likely that such idiotype variant tumor cells were already present before the monoclonal antibody treatment, but were allowed to proliferate after the selective removal of the idiotype positive tumor cells.

Active immunization of animals with syngeneic tumor derived Ig or its subfragments elicits the production of anti-id antibodies and induces protection against a subsequent exposure to tumor cells. In most cases, however, a tumor elicits a response which is too weak or which appears too late to be of lasting therapeutic value. Therefore, either modified id-Ig or strong non-physiological adjuvants were needed. The use of non-physiological immunogens or non-specific activators is highly undesirable for use with human patients because of side effects, including the possibility of inducing a polyclonal $\beta$ cell response which could lead to the development of autoimmune disease.

There thus exists a long-felt need for enhancing the immunogenicity of vaccines. Preferably, any adjuvants used in connection with such immunization should provide an immune response of sufficient strength to be therapeutically useful. In particular there exists a need for a means to effect active immunization of the tumor host with syngeneic Ig so as to elicit an effective polyclonal response. The present invention satisfies this need and provides related advantages as well.

SUMMARY OF THE INVENTION

The invention provides a use of dendritic cells previously pulsed with the idiotype protein of interest for the production of a medicament for inducing an effective immune response to pathogenic lymphocytes. In one embodiment, such a use for the production of a medicament for the active immunization of a mammal against lymphoma is provided. This embodiment provides a use of idiotype pulsed dendritic cells obtained by exposing dendritic cells to idiotype Ig for the production of an injectable medicament for inducing immunity against lymphoma cells in a mammal. In another embodiment, the invention relates to the use of both idiotypic cells and pulsed dendritic cells.

## BRIEF DESCRIPTION OF THE DRAWINGS

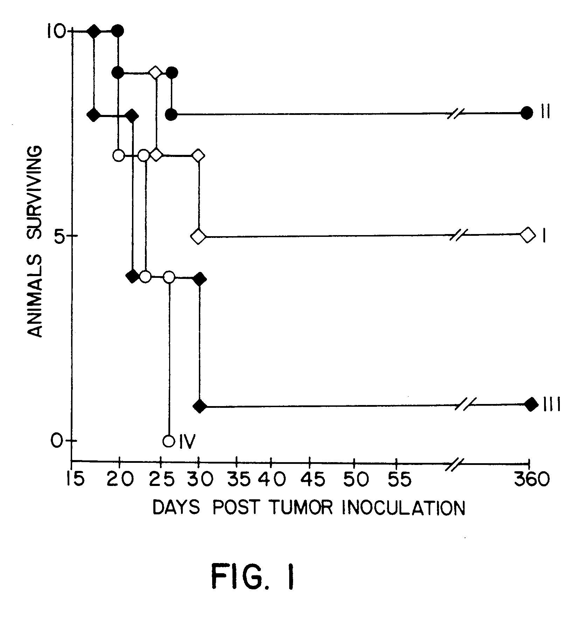

Figure 1. Tumor immunity induced immunization with idiotype-KLH conjugates. $C_3H$/He mice were immunized at weekly intervals with KLH conjugates of 38C13 IgM (-˙-˙- two injections: group II, -0-0- three injections: group I, -0-0- 1 injection: group III) or control IgM (-0-0- two injections: group IV). One week after the last immunization, mice were inoculated with $10^2$ 38C13 tumor cells. The numbers correspond to the experimental groups of Table I.

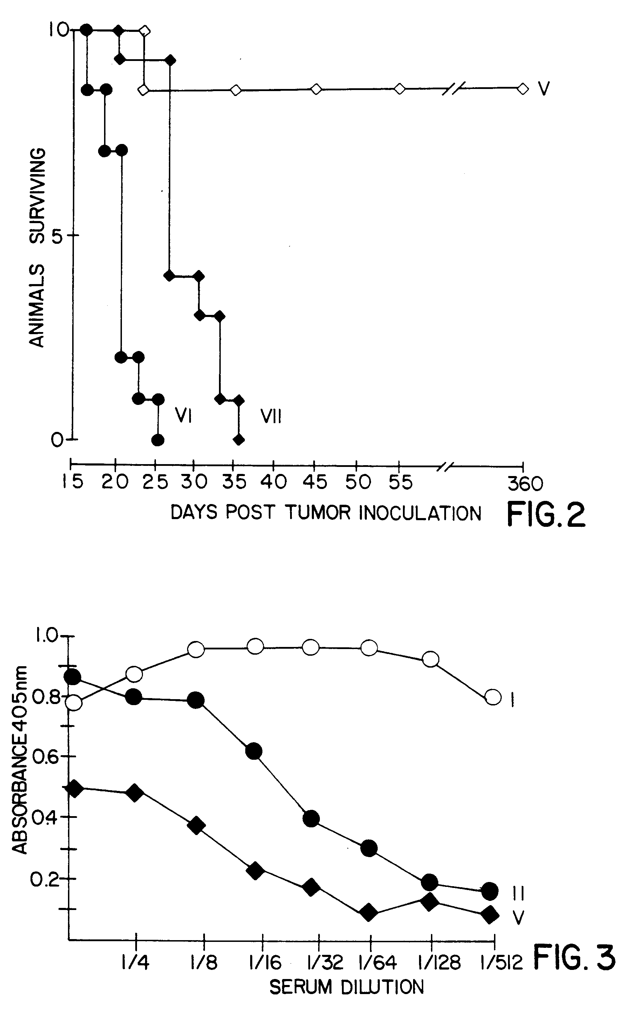

Figure 2. Effect of immunization with idiotype IgM pulsed dendritic cells (DC) on survival of mice after tumor inoculation. C3H/He mice were immunized with 38IC13 IgM-DC (-0-0- : group V), or control IgM-DC (-˙-˙- : group VI) or soluble Idiotype protein (-♦-♦-: group VII) at day 28 before tumor cell ($10^2$) challenge. Mice received one boost of soluble IgM at day -7. The numbers correspond to the experimental groups of Table I.

Figure 3. Comparison of syngeneic anti-idiotype antibodies induced by immunization with 38C13-KLH in Freund's adjuvant and by 38C13 pulsed DC. Sera from mice three (-0-0- : group I) or two (-˙-˙- : group II) times immunized with 38C13 KLH conjugates emulsified in Freund's adjuvant or with 38 C13 pulsed DC (-♦-♦-: group V) were added to wells pre-coated with 38C13 IgM. The sera were diluted over 8 wells. Bound antibody was detected by addition of enzyme labeled goat anti-mouse IgG.

The invention relates to enhancing the effect of immunization. Such an enhancement is of particular usefulness for immunization in situations where an effective immune response is difficult to elicit, as with many tumor markers. However, the enhancement is also applicable in conjunction with immunization against various pathogens.

Recently, a dramatic enhancement of an antiviral immune response by rouse dendritic cells pulsed in vitro with virus or with polyclonal anti-idiotype antibodies ($Ab_2$) was reported Francotte and Urbain PNAs 82:8149 (1985). Dendritic cells (DC) have been shown to be strong stimulators of immune responses to antigens attached to their cell surface. The antigen pulsed DC primes the resting T lymphocytes which then deliver the necessary help to antigen-specific B lymphocytes.

Thielemans et al., J. Immunol. 133:495-501 (1984) describes various strategies for producing anti-idiotype antibodies against human B-cell lymphomas. In addition, it is noted that idiotype immunoglobulins are present on the cell surface of such lymphomas.

The present invention involves the unexpected determination that mouse DC pulsed in vitro with idiotype protein from pathogenic lymphocytes can replace the immunogenic carrier and the non-physiological adjuvant previously thought to be required to elicit an effective immune response to pathogenic lymphocytes. As used herein, the term "pathogenic lymphocytes" refers either to unregulated malignant lymphocytes or to lymphocytes mounted in an autoimmune response. The term idiotype protein from a pathogenic lymphocyte refers to an immunoglobulin or a fragment of an immunoglobulin (FAB) bearing an idiotypic epitope.

Protection against a subsequent tumor cell dose can be obtained by pre-immunization with syngeneic idiotype conjugated to an immunogenic carrier and emulsified in Freund's adjuvant or with in vitro idiotype-pulsed DC. The control groups treated with the same number of DC pulsed with an irrelevant IgM or immunized with the same dose of soluble syngeneic 38C13 IgM, did not show a prolonged survival. These data clearly indicate the enhancing effect of dendritic cells and the idiotype specific suppression of tumor growth.

$C_3H$/He mice were immunized with idiotypic immunoglobulin M (IgM) from the syngeneic 38C13 lymphoma. Conjugation to an immunogenic carrier protein (keyhole limpet hemocyanin; KLH) and a strong non-physiological immune stimulator (complete Freund's adjuvant; CFA) was required to obtain an idiotype specific humoral and cellular immunity and protection against a lethal tumor cell challenge. However, when dendritic cells were used for idiotype presentation, neither immunogenic carrier nor adjuvant were needed. Dendritic cells, having been pulsed in vitro with unmodified idiotype protein and re-injected into the animals, were able to induce significant resistance to subsequent tumor inoculation. Alternatively FAB fragments or synthetic peptides bearing an idiotype epitope could be used for inoculation. Idiotypic specific T-lymphocytes which proliferated in response to native 38C13 idiotype (id) as well as cytotoxic T lymphocytes were observed in both groups. The cellular immune response was stronger in the dendritic cell-treated animals than in case of 38C13-KLH and complete Freund's adjuvant treatment. Both immunization methods resulted in long-term survivors without tumor cell escape caused by emergence of idiotype variants or tumor cell dormancy. Remarkably, after one year, 80% of the mice were still alive.

Serum analysis of immunized animals showed that the protective effect was not correlated in a simple way to the serum anti-idiotype titer. High levels of anti-idiotype antibodies induced by hyper-immunization with Freund's adjuvant could have modulated the surface Ig of the tumor cells which then could escape destruction by id-specific T cells.

T cells that proliferated in the presence of idiotype proteins could be demonstrated even more than 3 months after the immunization. These cells, although their phenotype is not known, had a cytotoxic effect on the syngeneic tumor cells.

The following examples are intended to illustrate but not limit the invention.

## EXAMPLE I

### Preparation of Dendritic Cells

Dendritic cells (DC) were isolated using the method described by Steinman and Cohen, J. Exp. Med. 139:380-397 (1974). Briefly a suspension of spleen cells, free of aggregates or clusters, was suspended in a solution of bovine serum albumin (BSA) (p = 1,082 g/cm$^3$); Fraction V, (Sigma Chemical Co., St. Louis, MO), at a concentration of 1 X 10$^8$ cell per ml. A low density BSA solution (p = 1,060 g/cm$^3$) was layered on top. The tubes were spun to equilibrium at 10,000 g for 30 minutes at 4°C. Floating cells were harvested and washed twice in RPMI 1640 medium. The cells were resuspended in complete medium (RPMI 1640, 5% FCS, 5 X 10$^{-5}$ M 2-mercapto-ethanol, penicillin, streptomycin, minimal essential amino acids, and sodium pyruvate) and transferred to plastic petri dishes at a concentration of 1 X 10$^5$ cell/ml for 3 hours at 37°C - 5% $CO_2$. Non-adherent cells were removed by gentle pipetting and the adherent cells were kept for another 16 hours in complete medium. The supernatant containing non-adherent low density cells was used as the source of DC. The contaminating macrophages were removed during antigen pulsing, since the macrophages tended to re-adhere to the plastic surface while the DC remained non-adherent. The purity of the final cell preparation was examined by scanning EM, transmission EM, immunofluorescence and acridine orange staining. Goat anti-mouse Ig labelled with FITC, and anti-Thy-1.2 labelled to biotin (Becton Dickinson), were used to characterize B and T cells respectively.

Dendritic cells were resuspended in complete medium at a concentration of 5 X 105 cells per ml in 24 well flat-bottomed plates (1 ml per well). Fifty microgram of purified 38C13 IgM (kappa) idiotype protein or an irrelevant mouse IgM (kappa) (ABPC - Sigma Chemical Co., St. Louis, MO) was added. The plates were kept at 37°C, in 5% $CO_2$ for 4 to 5 hours. Cells were washed several times in sterile PBS and resuspended at 2.5 X 10$^5$ cell per ml.

## EXAMPLE II

### Syngeneic Immunization With Idiotypic Ig

$C_3H/He$ mice were obtained from the Laboratory Animal Center of the Catholic University of Leuven. Balb/c and F1($C_3H/He$ x Balb/c) mice were bred at the Laboratory Animal Facility at the Free University of Brussels (VUB). 38C13 is a carcinogen (DMBA) induced B cell tumor of $C_3H$ origin. These tumor cells and the in vitro adapted cell line used in this study express IgM (kappa) on the cell membrane but do not secrete large amounts of Ig. An idiotype IgM (kappa) secreting cell line has been obtained by fusion of the tumor cells with a non-secretory myeloma cell line (P3 X 63 Ag 8.653).

Four groups of 10 mice each were immunized with 50 μg idiotypic 38C13 IgM or unrelated ABPC IgM cross-linked to KLH, emulsified in Freund's complete adjuvant (CFA), incomplete adjuvant (ICFA) or PBS, following a schedule shown in Table 1. One week after the last injection 38C13 tumor cells (10$^2$ cells/mouse) were injected intraperitoneally. This dose is lethal to 100% of non-treated animals by day 30. The presence of tumor and the day of death were recorded.

Figure 1 shows the protective effect of immunization on survival in a typical experiment. The most effective immunization schedule consisted of 2 administrations of idiotypic IgM, once in CFA and once in ICFA (group II). These findings were reproducible. There were no long-term survivors when either an irrelevant IgM-KLH conjugate (group IV) or unconjugated idiotypic IgM (group VII) were used.

## TABLE I

| IMMUNIZATION SCHEDULE | | | | |
|---|---|---|---|---|
| +Time | I* | II | III | IV |
| Day 28 | - | - | - | - |
| Day 21 | 38C-KLH/CFA i.p. | - | - | - |
| Day 14 | 38C-KLH/ICFA i.p. | 38C-KLH/CFA i.p. | - | ABPC-KLH/CFA i.p. |
| Day 7 | 38C-KLH/PBS i.v. | 38C-KLH/ICFA i.p. | 38C-KLH/CFA i.p. | ABPC-KLH/ICFA i.p. |

*Experimental group consisting of 10 mice.

+Day before the i.p. injection of tumor cells ($10^2$) at day 0.

EXAMPLE II

Immunization with Idiotypic IgM Pulsed Dendritic Cells

These groups of 10 each of $C_3H$/He mice were injected intraperitoneally with 5 X $10^4$ IgM pulsed dendritic cells prepared as in Example I, according to the immunization schedule of Table II.

## TABLE II

| +Time | V | VI | VII |
|---|---|---|---|
| Day 28 | DC-38C13 i.v. | DC-ABPC i.v. | 38C13 i.v. |
| Day 21 | - | - | - |
| Day 14 | - | - | - |
| Day 7 | 38C13 i.v | ABPC i.v. | 38C13 i.v. |
| Experimental group consisting of 10 mice. | | | |

+Day before the i.p. injection of tumor cells ($10^2$) at day 0.

One hundred 38C13 cells were injected intraperitoneally one week after the last injection of antigen. The presence of tumor cells and the day of death were recorded.

The protective effect of immunization with idiotypic IgM pulsed dendritic cells is shown in Figure 2. A single injection of idiotype pulsed DC's followed by a boost of soluble idiotype protein resulted in the same survival after tumor passage as in the experiments using 38C13-KLH conjugates and CFA. Control immunized animals (DC pulsed with irrelevant IgM or idiotype protein alone) did not show any protection.

EXAMPLE IV

Assessment of Humoral and Cellular Immunity

To assess the role of humoral immunity, the levels of syngeneic anti-idiotypic antibodies were measured by an IgG specific ELISA prepared as follows.

Balb/c mice were immunized with purified idiotype protein cross-linked with keyhole limpet hemocyanin (KLH) (Calbiochem-Behring, Hoechst) with glutaraldehyde according to the method of Maloney, et al., Hybridoma 4:191-209 (1985), which is incorporated herein by reference. The spleen cells were hybridized to the P3 x 63 Ag 8.653 myeloma cell line. The monoclonal antibodies E4 and 8E3 were strongly reactive with 38C13 IgM(kappa), were not inhibitable by normal $C_3H$ serum and did neither bind to normal spleen cells nor to purified IgM myeloma proteins. A monoclonal anti-idiotype antibody S5A8, of $C_3H$ origin and a rat monoclonal antibody R7D7 were purified from ascites fluid by double precipitation with ammonium sulfate (40%) were utilized. Biotin labelling of the antibodies was performed according to methods well known in the art.

Before the tumor cell injection, the mice were bled by puncture of the retro-orbital plexus. Sera from individual mice of the same experimental group were pooled. Syngeneic anti-idiotype antibodies were detected by an ELISA assay as described.

The results in Figure 3 indicate high levels of anti-id antibodies in mice immunized with 38C13-KLH and CFA. Much lower levels were detected in sera from animals immunized with DC's, indicating that there was no clear correlation between antibody levels and survival. Three injections with IgM-KLH in CFA resulted in a higher serum level of anti-id antibodies but a lower survival rate (Figure 1).

Cellular immunity was determined by detecting idiotypic specific T lymphocytes in the spleen of long-term survivors of both immunization approaches. T cell-enriched splenic cells were cultured in the presence of soluble idiotype protein or idiotypic IgM coupled to sepharose beads for 3 days. Control wells contained splenocytes in IL2 containing medium 10% (v/v) of supernatant of rat spleen cell culture containing 4 $\mu$g/ml concanavalin A for 24 hours, or an irrelevant IgM protein. A suspension of splenic cells from surviving animals was transferred to plastic petri dishes (80 mm), pre-coated with 0.2% BSA, at $10^7$ cells/ml in 3 ml complete medium at 37°C for 1 hour(27). Non-adherent cells were transferred to plates pre-coated with rabbit anti-mouse (kappa) (10 $\mu$g/ml) and placed at 4°C for 1 hour. A second panning was performed on plates pre-coated with goat mouse Ig (Tago, Burlingame, CA). Recovery after this double panning procedure generally was 25 to 30% of the nucleated cells. In later experiments, T cells were enriched by binding to nylon wool by methods well known in the art. B cell contamination was examined by immunofluorescence using fluorescent goat anti-mouse Ig antibodies (Tago). The B cell fraction was usually 5 to 7%. The cell suspension enriched for T cells was placed in round bottomed wells (200 $\mu$l/well, 5 X $10^5$ cells/ml). Twenty $\mu$l of stimulating agent (50 $\mu$g/ml) was added to the wells. After 3 days of culture, 1.5 $\mu$Ci of [methyl $^3$H]-thymidine was added to each well. After this 18 hour pulse, cells were harvested on glass fiber filters and incorporated radioactivity measured by scintillation counting. All measurements were performed in quadruplicate and the data are expressed as the mean cpm ± SEM.

The results of the in vitro stimulation are shown in Table II. T cells from mice treated with DC-38C13 responded better to the idiotype protein than mice treated with idiotype KLH conjugates in CFA, or control mice.

## PROLIFERATIVE RESPONSES IN LONG-TERM
## SURVIVING ANIMALS[*]
## [$^3$H] THYMIDINE INCORPORATION (cpm ± SEM

| | 38C13 IgM | 38C13-Sepharose | IL2 | Unrelated IgM |
|---|---|---|---|---|
| DC-38C13 | 23,451 ± 1286[+§] | 6,787 ± 376[§] | 10,071 ± 563[§] | 1,414 ±160 |
| id.KLH in CFA | 1,084 ± 127 | 11,271 ± 898[+§] | 2,166 ± 527[#] | 4,805 ± 363[''] |
| −[†] | 1,453 ± 661 | 2,996 ± 243 | 850 ± 302 | 2,195 ± 395 |

Table 2. [*]Enriched splenic T cells were cultured in vitro during 3 days in the presence of 38C13 IgM. 38C13 IgM coupled to Sepharose beads, IL2 containing medium or soluble unrelated IgM. Stimulation was measured by the degree of $^3$H-thymidine incorporation during the last 18 hours of culture. [†]Normal C$_3$H/He mice were used to purify unprimed T cells. [*]Significant at $p < 0.001$ according to t-test with unrelated IgM. [§]Significant at $p < 0.001$ according to t-test compared with unprimed T cells. [#]$p > 0.05$ according to t-test compared with unprimed T cells. ['']Significant at $p < 0.002$ according to t-test compared with unprimed T cells.

The T cell enriched spleen cells were also used in a conventional cell mediated cytotoxicity assay as follows:

Tumor cells were labelled with L-[4,5$-^3$H] leucine (Amersham) and were placed in U-bottomed microwells at $1 \times 10^4$ target cells (100 $\mu$l). Effector cells (enriched splenic T cells from surviving animals) were added at a ratio of 100/1, 50/1, 25/1, 6.25/1, 3/1, 1.5/1 in triplicate, the final volume being 200 $\mu$l. The cells were sedimented by gentle centrifugation and the plates were incubated at 37°C in a moist atmosphere containing 5% $CO_2$ for 16 hours. Fifty microliters of supernatant was used to count the released radioactivity. Spontaneous and maximum release was determined by adding 100$\mu$l complete medium or 100 $\mu$l 0.1% NP40 instead of T cells. Specific cytotoxicity was calculated according to the following formula:

$$\% \text{ specific release} = \frac{\text{experimental release} - \text{spontaneous release}}{\text{maximum release} - \text{spontaneous release}} \times 100$$

Cytotoxicity by immune lymphocytes was measured the day of T cell isolation and after a 3 day stimulation in vitro with 38C13-coupled sepharose beads. Unstimulated T cells were not able to lyse the 38C13 target cells. However, after stimulation in vitro, specific lysis of 38C13 tumor cells by DC-38C13 primed T cells was 21% versus 11% by 38C13-KLH stimulated cells (at an effector target ratio of 100/1 in a 16 hour incubation assay).

EXAMPLE V

Residual Tumor Cells

The presence of residual tumor cells in the spleen of long-term survivors was traced out by immunohistology and in vitro culture. Cryostat sections were fixed in acetone and stained with the monoclonal antibodies followed by streptavidin-horseradish peroxidase and diaminobenzidine tetrahydrochloride. Frozen sections of the spleen from long-term surviving animals were stained with biotinylated monoclonal anti-idiotype antibodies. These antibodies had previously been tested on tumor invaded spleen-sections where they stain both surface and cytoplasmic idiotypic IgM. No residual tumor cells could be detected in serial sections of the spleen of long-term survivors. B lymphocytes isolated from the spleen were cultured in vitro in enriched and conditioned medium. Although the 38C13 tumor cells used in this study are adapted to in vitro growth, none of the cultures showed outgrowth of tumor cells. Finally, B cells were transferred to irradiated syngeneic naive animals. No growth of tumor was observed during the 6 month observation time. These data indicate that no residual tumor cells remained in spleens of long term survivors.

EXAMPLE VI

Alternative Preparation of Dendritic Cells

Spleens were perfused with a solution of 100 units/ml collagenase (CLS III, Worthington, Cat. Nr 4182, 1g/vial.) in 10 X Hanks Balanced Salt Solution (HBSS), (diluted from 10 X HBSS; Gibco Cat. Nr. 310-4065A/J.), (pH 7.2. adjusted with sterile GIBCO 7.5% bicarbonate). The cells were collected in a tube containing RPMI medium with 10% FCS. The remaining debris were incubated for 15 minutes in a solution of 400 units/ml collagenase, in the incubator. The solution was pipetted about 50 times to collect most of the cells. All the cells were pooled and pelleted.

Bovine Serum Albumin was prepared as follows: BOVUMINAR COHN FRACTION V powder (Armour Pharmaceutical Company, Kankakee, Illinois) was kept at 4°C in a dessicator. In a glass beaker (closed with aluminum foil), 65 ml distilled water, 186 ml PBS, 29 ml 1M NaOH and 107g BSA were combined and kept at 4°C for 24 hours without stirring. Density was checked in a refractometer and maintained between 1.3855-1.3865. If the solution was too dense, it was stirred very slowly and cold PBS was added. It was filtered and kept at 4°C. The pellet was resuspended in a solution of cold BSA and centrifuged at 10,000 g; about 2 mls of serum-free medium was added on the top of the gradient. Low density cells were collected and represented about 10% of spleen cells.

The cells were cultured in 10% FCS-containing medium for 2 hours in 100 mm culture dishes. Non-adherent cells were washed out by vigorous pipetting (15 minutes per plate). Cells were again incubated for 1 hour in serum-free medium and non- adherent cells were removed by gentle pipetting. The remaining adherent cells were cultured overnight in 10% FCS-containing RPMI. The next day, the non-adherent cells were collected by gentle pipetting. 2 X $10^5$ to 5 X $10^5$ dendritic cells were obtained per spleen.

Although the invention has been described with reference to the presently-preferred embodiment, it should be understood that the invention is limited only by the following claims.

**Claims**
**Claims for the following Contracting States : AT, BE, CH, DE, DK, FR, GB, IT, LI, LU, NL, SE**

1. Use of idiotype pulsed dendritic cells obtained by exposing dendritic cells in vitro to idiotype protein from a pathogenic lymphocyte for the production of an injectable medicament for inducing an effective immune response in a mammal to said pathogenic lymphocytes expressing an idiotype protein on the cell membrane.

2. The use of claim 1 wherein said pathogenic lymphocytes are tumor cells.

3. The use of claim 2 wherein said tumor cells are lymphoma cells.

4. The use of claim 3 wherein said lymphoma cells are B lymphoid cells.

5. The use of claims 1 to 4 wherein said idiotype protein is immunoglobulin.

6. The of claim claims 1 to 4 wherein said idiotype protein is a fragment of an immunoglobulin bearing an idiotype.

7. The use of claims 1 to 6 wherein said mammal is a human.

8. Idiotype pulsed dendritic cells comprising dendritic cells which have been exposed in vitro to idiotype immunoglobulins.

**Claims for the following Contracting States : ES, GR**

1. A method for the production of injectable medicament for inducing an effective immune response in a mammal to pathogenic lymphocytes expressing an idiotype protein on the cell membrane by obtaining idiotype pulsed dendritic cells through exposure of dendritic cells in vitro to idiotype protein from said pathogenic lymphocyte and formulating the same as an injectable medicament .

2. The method of claim 1 wherein said pathogenic lymphocytes are tumor cells.

3. The method of claim 2 wherein said tumor cells are lymphoma cells.

4. The method of claim 3 wherein said lymphoma cells are B lymphoid cells.

5. The method of claims 1 to 4 wherein said idiotype protein is immunoglobulin.

6. The method of claims 1 to 4 wherein said idiotype protein is a fragment of an immunoglobulin.

7. The method of claims 1 to 6 wherein said mammal is a human.

8. A method for the production of idiotype pulsed dendritic cells by exposing dendritic cells in vitro to idiotype immunoglobulins.

**Patentansprüche**
**Patentansprüche für folgende Vertragsstaaten : AT, BE, CH, DE, DK, FR, GB, IT, LI, LU, NL, SE**

1. Verwendung von Idiotyp-gepulsten dendritischen Zellen, welche durch Exposition von dendritischen Zellen in vitro mit Idiotyp-Protein von einem pathogenen Lymphozyten erhalten wurden, zur Herstellung eines injizierbaren Medikaments zur Auslösung einer effektiven Immunreaktion in einem Säuger gegen die pathogenen Lymphozyten, die ein Idiotyp-Protein auf der Zellmembran exprimieren.

2. Verwendung nach Anspruch 1, bei der die pathogenen Lymphozyten Tumorzellen sind.

3. Verwendung nach Anspruch 2, bei der die Tumorzellen Lymphomzellen sind.

4. Verwendung nach Anspruch 3, bei der die Lymphomzellen B-lymphoide Zellen sind.

5. Verwendung nach den Ansprüchen 1 bis 4, bei der das Idiotyp-Protein ein Immunglobulin ist.

6. Verwendung nach den Ansprüchen 1 bis 4, bei der das Idiotyp-Protein ein Fragment eines Immunglobulins ist, das einen Idiotyp trägt.

7. Verwendung nach den Ansprüchen 1 bis 6, bei der der Säuger ein Mensch ist.

8. Idiotyp-gepulste dendritische Zellen, umfassend dendritische Zellen, die in vitro mit Idiotyp-Immunglobulinen exponiert worden sind.

**Patentansprüche für folgende Vertragsstaaten : ES, GR**

1. Verfahren zur Herstellung eines injizierbaren Medikaments zur Auslösung einer effektiven Immunreaktion in einem Säuger gegen pathogene Lymphozyten, die auf der Zellmembran ein Idiotyp-Protein exprimieren, durch Gewinnung von Idiotypgepulsten dendritischen Zellen durch Exposition von dendritischen Zellen in vitro mit Idiotyp-Protein von dem pathogenen Lymphozyten und Formulierung derselben zu einem injizierbaren Medikament.

2. Verfahren nach Anspruch 1, bei dem die pathogenen Lymphozyten Tumorzellen sind.

3. Verfahren nach Anspruch 2, bei dem die Tumorzellen Lymphomzellen sind.

4. Verfahren nach Anspruch 3, bei dem die Lymphomzellen B-lymphoide Zellen sind.

5. Verfahren nach den Ansprüchen 1 bis 4, bei dem das Idiotyp-Protein ein Immunglobulin ist.

6. Verfahren nach den Ansprüchen 1 bis 4, bei dem das Idiotyp-Protein ein Fragment eines Immunglobulins ist.

7. Verfahren nach den Ansprüchen 1 bis 6, bei dem der Säuger ein Mensch ist.

8. Verfahren zur Herstellung von Idiotyp-gepulsten dendritischen Zellen durch Exposition von dendritischen Zellen in vitro mit Idiotyp-Immunglobulinen.

**Revendications**
**Revendications pour les Etats contractants suivants : AT, BE, CH, DE, DK, FR, GB, IT, LI, LU, NL, SE**

1. Utilisation de cellules dendritiques stimulées par un idiotype obtenues en exposant des cellules dendritiques in vitro à une protéine idiotypique issue d'un lymphocyte pathogène pour produire un médicament injectable destiné à induire une réponse immunitaire efficace chez un mammifère vis-à-vis desdits lymphocytes pathogènes exprimant une protéine idiotypique sur la membrane cellulaire.

2. Utilisation selon la revendication 1 dans laquelle lesdits lymphocytes pathogènes sont des cellules tumorales.

3. Utilisation selon la revendication 2 dans laquelle lesdites cellules tumorales sont des cellules de lymphome.

4. Utilisation selon la revendication 3 dans laquelle lesdites cellules de lymphome sont des cellules lymphoïdes B.

5. Utilisation selon l'une des revendications 1 à 4 dans laquelle ladite protéine idiotypique est une immunoglobuline.

6. Utilisation selon l'une des revendications 1 à 4 dans laquelle ladite protéine idiotypique est un fragment d'une immunoglobuline portant un idiotype.

7. Utilisation selon l'une des revendications 1 à 6 dans laquelle ledit mammifère est l'homme.

8. Cellules dendritiques stimulées par un idiotype comprenant des cellules dendritiques qui ont été exposées in vitro à des immunoglobulines idiotypiques.

**Revendications pour les Etats contractants suivants : ES, GR**

1. Procédé de production d'un médicament injectable destiné à induire une réponde immunitaire efficace chez un mammifère vis-à-vis de lymphocytes pathogènes exprimant une protéine idiotypique sur la membrane cellulaire en obtenant des cellules dendritiques stimulées par un idiotype par exposition in vitro de cellules dendritiques à une protéine idiotypique issue dudit lymphocyte pathogène et en les formulant sous forme d'un médicament injectable.

2.  Procédé selon la revendication 1 dans laquelle lesdits lymphocytes pathogènes sont des cellules tumorales.

3.  Procédé selon la revendication 2 dans laquelle lesdites cellules tumorales sont des cellules de lymphome.

4.  Procédé selon la revendication 3 dans laquelle lesdites cellules de lymphome sont des cellules lymphoïdes B.

5.  Procédé selon l'une des revendications 1 à 4 dans laquelle ladite protéine idiotypique est une immunoglobuline.

6.  Procédé selon l'une des revendications 1 à 4 dans laquelle ladite protéine idiotypique est un fragment d'une immunoglobuline.

7.  Procédé selon l'une des revendications 1 à 6 dans laquelle ledit mammifère est l'homme.

8.  Procédé de production de cellules dendritiques stimulées par un idiotype en exposant in vitro des cellules dendritiques à des immunoglobulines idiotypiques.

FIG. 1

FIG.2

DAYS POST TUMOR INOCULATION

FIG. 3

SERUM DILUTION